Europäisches Patentamt

European Patent Office

Office européen des brevets

(19)

(11) Veröffentlichungsnummer: **0 114 977**
**B1**

(12) **EUROPÄISCHE PATENTSCHRIFT**

(45) Veröffentlichungstag der Patentschrift:
22.06.88

(51) Int. Cl.⁴: **A 61 N 5/06**, A 61 H 33/06

(21) Anmeldenummer: **83111995.3**

(22) Anmeldetag: **30.11.83**

(54) **Thermisch-solare Behandlungscabine für Personen und/oder Tiere zur Behandlung durch die Wirkung der Sonnenstrahlung, insbesondere für solare Sauna-Bäder.**

(30) Priorität: **24.12.82 IT 6852182**

(43) Veröffentlichungstag der Anmeldung:
**08.08.84 Patentblatt 84/32**

(45) Bekanntmachung des Hinweises auf die Patenterteilung:
**22.06.88 Patentblatt 88/25**

(84) Benannte Vertragsstaaten:
**AT BE CH DE FR GB LI LU NL SE**

(56) Entgegenhaltungen:
**DE - A - 2 624 632**
**DE - A - 2 943 842**
**US - A - 1 772 219**
**US - A - 2 493 328**
**US - A - 2 653 612**
**US - A - 4 277 855**

(73) Patentinhaber: **Costantino, Giovanni, Via Guido Cane 29, I-12050 Valle Talloria di Diano d'Alba (Cuneo) (IT)**

(72) Erfinder: **Costantino, Giovanni, Via Guido Cane 29, I-12050 Valle Talloria di Diano d'Alba (Cuneo) (IT)**

(74) Vertreter: **Porsia, Bruno et al, c/o Succ. Ing. Fischetti & Weber Via Caffaro 3/2, I-16124 Genova (IT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine thermisch-solare Behandlungskabine zur Behandlung von Personen und/oder Tieren, die sich im Innenraum der Behandlungskabine befinden, durch Einwirkung der Sonnenstrahlen, insbesondere für solare Sauna-Bäder, mit Belüftungsöffnungen und einem von unten nach oben gegen das Innere der Kabine geneigten Glasfenster. Eine Behandlungskabine der genannten Art ist aus US-A-1.772.219 bekannt.

Der Erfindung liegt die Aufgabe zugrunde, eine Kabine der genannten Art zu schaffen, die eine wirksame Behandlung von Personen und/oder Tieren durch direkte und/oder indirekte Einwirkung der Sonnenstrahlen vor allem zu Heilzwecken, wie z.B. zur heliotherapeutischen Behandlung, aber auch zu allgemeinen Zwecken der Hygiene und der Körperpflege sowie zur Verbesserung und Rationalisierung der Zucht von Haustieren und/oder in der Gefangenschaft lebenden Tieren. Dabei soll gemäß der Erfindung die Behandlungskabine eine rationelle Ausnutzung der Wirkung der Lichtstrahlen (insbesondere der ultravioletten und infraroten Strahlen) und/oder der Wärmeenergie der Sonnenstrahlen unter steuerbaren Bedingungen und mit hohem Wirkungsgrad ermöglichen, und im einzelnen in dem zu behandelnden Subjekt sowohl die Pigmentierung der Haut als auch die Schweißausscheidung durch die Schweißdrüsen fördern; dadurch soll dem zu behandelnden Subjekt die Möglichkeit geboten sein, in ein und demselben Raum mit ein und derselben Behandlung die wohltuenden und heilbringenden Resultate zu erreichen, die sonst durch getrennte Behandlungen und in verschiedenen Örtlichkeiten (wie Solarium, Sauna u.a.) erzielt werden können, oder aber die Alternative geboten werden, jeweils eine gewünschte spezifische Behandlungsart stets im selben Behandlungsraum zu wählen.

Außerdem soll die erfindungsgemäße Behandlungskabine bequem verfahrbar und/oder orientierbar sein, damit deren Standort und/oder Stellung gegenüber der Sonneneinstrahlung nach Bedarf geändert werden können.

Endlich soll die erfindungsgemäße Behandlungskabine einfach in ihrem Aufbau und in der Bedienung sowie zuverlässig und sicher in ihrer Benutzung sein.

Dieser Aufgabenstellung entsprechend ist die erfindungsgemäße Behandlungskabine dadurch gekennzeichnet (Patentanspruch 1), daß zur rationellen Ausnützung der Wirkung der Lichtstrahlen, insbesondere der ultravioletten und infraroten Sonnenstrahlen, unter steuerbaren Bedingungen und mit hohem Wirkungsgrad:

a) ein Glasfenster sich auf einer Kabinenwand im wesentlichen über die ganze Höhe der Kabine erstreckt,

b) zwischen dem Rahmen des Glasfensters und dem Kabinenkörper ein allseitig dichter Verschluß vorgesehen ist,

c) die Öffnungsweite von Belüftungsöffnungen mit Hilfe von verstellbaren Abschlußmitteln zur Regelung des Luftdurchflusses einstellbar ist.

Mit besonderem Vorteil ist das oben nach innen geneigte Glasfenster im Neigungswinkel wahlweise einstellbar ausgebildet und besitzt Mittel, um das Fenster in der eingestellten Neigung festzusetzen (Anspruch 2).

Gemäß einem anderen Merkmal der Erfindung sind Abdichtungsmittel zwischen dem Glasfenster, der Tür und den zugeordneten Wandteilen der Behandlungskabine vorgesehen, um durch Witterungseinflüsse bedingtes Eindringen von Luft, Wasser, Staub und dergleichen zu verhindern (Anspruch 3).

Weitere Einzelheiten und Merkmale der Erfindung ergeben sich aus den Unteransprüchen 4 bis 6.

Die Erfindung wird nachstehend anhand der Zeichnung und der darin dargestellten Ausführungsformen näher erläutert. Es zeigen:

Fig. 1 bis 3 eine Seitenansicht, eine Ansicht von oben und eine Ansicht von unten eines ersten, vereinfachten Ausführungsbeispiels der erfindungsgemäßen Behandlungskabine;

Fig. 4 und 5 jeweils eine Ansicht in Richtung des Pfeiles F bzw. F1 der Fig. 1;

Fig. 6, 7 und 8 eine Seitenansicht, eine Ansicht von oben und eine Ansicht in Richtung des Pfeiles F2 der Fig. 6 einer zweiten Ausführungsform der erfindungsgemäßen Behandlungskabine;

Fig. 9 einen Längsschnitt längs der Linie IX–IX der Fig. 4, wobei das Glasfenster in seiner Neigung einstellbar ist;

Fig. 10 einen Querschnitt längs der Linie X–X der Fig. 9;

Fig. 11 die Einzelheit A der Fig. 9 in größerem Maßstab;

Fig. 12 einen teilweisen Querschnitt längs der Linie XII–XII der Fig. 11;

Fig. 13 die Einzelheit B der Fig. 9 in größerem Maßstab;

Fig. 14 eine Ansicht in Richtung des Pfeiles F3 der Fig. 13;

Fig. 15 die Einzelheit C der Fig. 9;

Fig. 16 eine Ansicht in Richtung des Pfeiles F4 der Fig. 15;

Fig. 17 einen Schnitt längs der Linie XVII–XVII der Fig. 9 in größerem Maßstab;

Fig. 18 eine dem Schnitt gemäß Fig. 17 ähnliche Darstellung, worin jedoch die Tür der Behandlungskabine gemäß Fig. 9 in halb offenem Zustand gezeigt ist;

Fig. 19 einen Querschnitt durch die Abdichtungsmittel zwischen Fenster, Tür und den zugeordneten Wandteilen der Kabine gemäß Fig. 9 in größerem Maßstab;

Fig. 20 einen Querschnitt längs der Linie XX–XX der Fig. 9 in größerem Maßstab;

Fig. 21 bis 23 eine Seitenansicht, eine Ansicht von oben und eine Ansicht von unten einer dritten Ausführungsform der erfindungsgemäßen Behandlungskabine;

Fig. 24 und 25 jeweils eine Ansicht in Richtung des Pfeiles F5 bzw. F6 der Fig. 21;

Fig. 26 einen Längsschnitt gemäß der Linie XXVI–XXVI der Fig. 24, wobei jedoch nur das Traggerippe der Behandlungskabine gemäß Fig. 24 samt der Bodenversteifung gezeigt ist;

Fig. 27 und 28 jeweils eine Ansicht von unten bzw. von oben des Traggerippes der Kabine gemäß Fig. 26, wobei jedoch die Bodenversteifung der größeren Klarheit wegen ausgelassen ist;

Fig. 29 eine Ansicht in Richtung des Pfeiles F7 der Fig. 26 des Tragrahmens für das Glasfenster der Kabine gemäß Fig. 24, in der dieser Tragrahmen der Klarheit halber nicht gezeigt ist;

Fig. 30 eine Ansicht von unten der Versteifung des die Kabine gemäß Fig. 26 tragenden Bodens;

Fig. 31 eine Detail-Ansicht in größerem Maßstab und im Querschnitt längs der Linie XXXI–XXXI der Fig. 26, wobei hier auch die im einzelnen in Fig. 26 nicht gezeigten Wandteile und deren Verbindungsmittel dargestellt sind;

Fig. 32 eine Ansicht in Richtung des Pfeiles F8 der Fig. 31;

Fig. 33 einen Schnitt gemäß der Linie XXXIII–XXXIII der Fig. 26, ähnlich dem Schnitt der Fig. 31;

Fig. 34 eine Ansicht in Richtung des Pfeiles F9 der Fig. 33;

Fig. 35 bis 37 Detail-Ansichten in größerem Maßstab und im Querschnitt verschiedener Ausführungsformen der Verbindungsmittel für aneinandergesetzte Wandteile;

Fig. 38 eine Ansicht einer vierten Ausführungsform der erfindungsgemäßen Behandlungskabine;

Fig. 39 und 40 jeweils eine Ansicht in Richtung des Pfeiles F10 bzw. F11 der Fig. 38, und

Fig. 41 eine Ansicht von oben der Behandlungskabine der Fig. 36.

Die in den Zeichnungen dargestellten Teile mit gleichartigem Aufbau und gleichartiger Funktion sind in den einzelnen Figuren mit gleichen Bezugszeichen versehen.

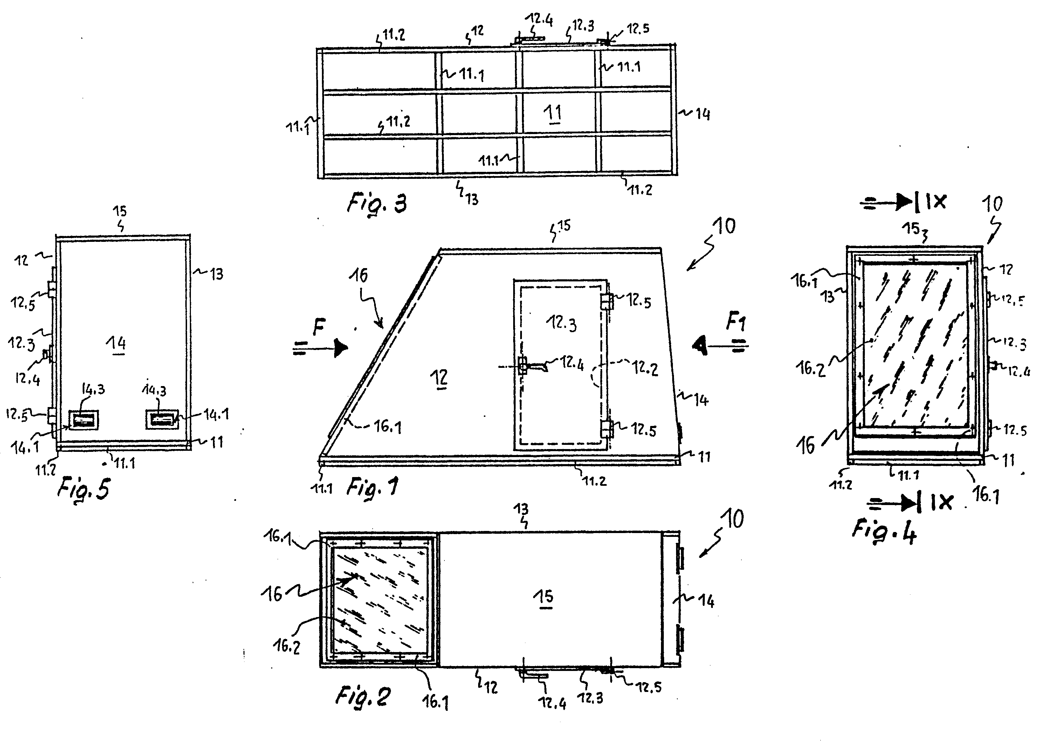

Die Fig. 1 bis 5 und 9 bis 20 zeigen eine vereinfachte erste Ausführungsform der erfindungsgemäßen, thermisch-solaren Behandlungskabine, die in ihrer Gesamtheit mit 10 bezeichnet ist.

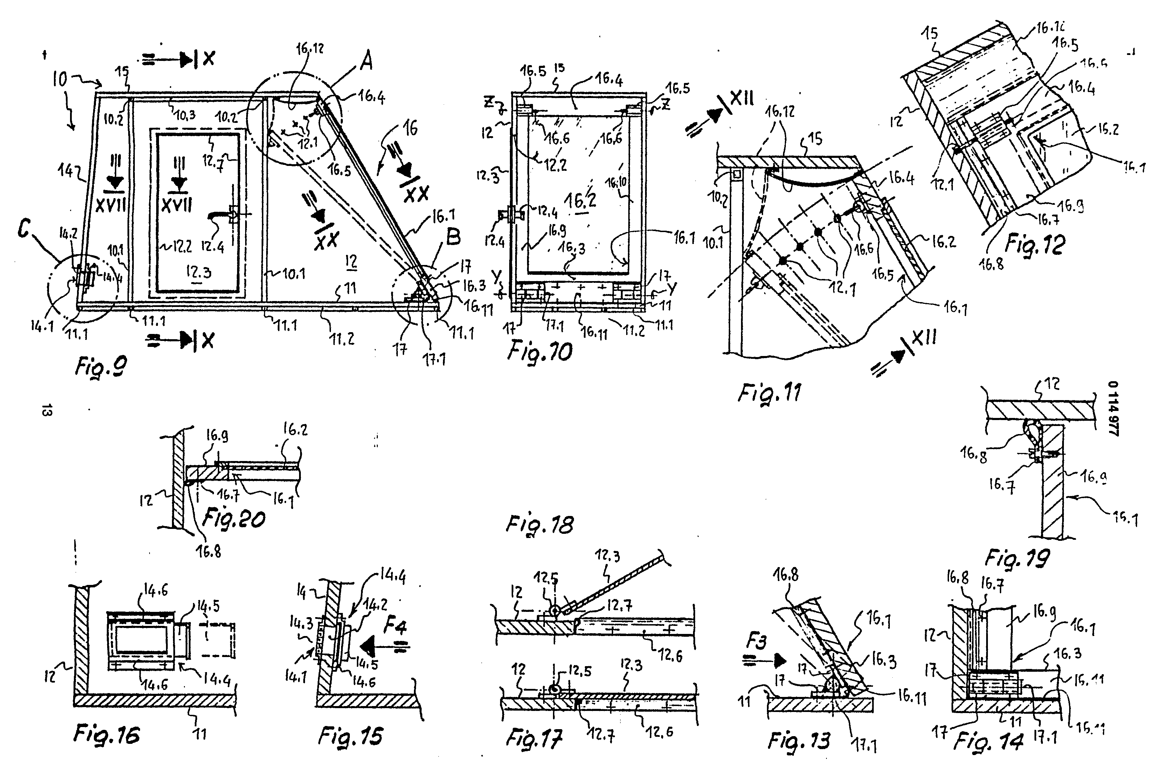

Die Kabine 10 umfaßt eine rechteckige Bodenplatte 11, senkrecht angeordnete, seitliche Längswände 12, 13 mit trapezförmigem, jedoch verschieden geneigte, nach oben zusammenlaufende Seiten aufweisendem Umfang, und eine rechteckige Querwand 14 zur Verbindung der weniger stark geneigten Seite jeder Längswand 12, 13 und der Bodenplatte 11. Ein steifes, rechteckiges Flachdach 15 ist mit den oberen Seiten der Längswände 12, 13 und der Querwand 14 verbunden, während die verbliebene Öffnung, die durch die nach freie Querseite der Bodenplatte 11 und des Daches 15 sowie die stärker geneigten Seiten der Längswände 12, 13 durch ein entsprechend bemessenes fest angeordnetes Fenster 16 mit rechteckigem Fensterrahmen 16.1 geschlossen wird, wobei dieses Fenster oben, d.h. der Neigung der erwähnten Seiten der Längswände entsprechend nach dem Innenraum der Kabine 10 hin geneigt ist (Fig. 1 bis 5). Die Strukturverbindung der Seitenwände 12, 13, 14 und des Daches 15 sowie die Verteilung der Aufbaulasten sind durch ein Traggerippe verwirklicht. Dieses aus senkrechten Stützen 10.1 und damit verbundenen Traversen 10.2 und Holmen 10.3 bestehende Traggerippe wirkt mit einem entsprechenden aus Traversen 11.1 und Holmen 11.2 bestehenden Trag- und Versteifungsrahmen für die Bodenplatte 11 zusammen (Fig. 3 und 9).

Wie aus den Fig. 9 und 10 hervorgeht, ist der Rahmen 16.1 des Glasfensters 16 gemäß einer abgewandelten Ausführung mit seiner untersten Traverse 16.3 mittels Scharniere 17 mit gemäß einer waagerechten Achse Y–Y quer zur Bodenplatte gerichteten Scharnierbolzen 17.1 an die Bodenplatte angelenkt. Dadurch ist der Fensterrahmen 16.1 um die erwähnte Achse Y–Y in einer senkrechten Längsebene der Kabine 10 schwenkbar. Außerdem besitzt der Fensterrahmen 16.1 an den Enden seiner obersten Traverse 16.4 Feststellriegel 16.5, deren Schieber 16.6 in ihren Führungen gemäß einer zur Achse Y–Y der Scharnierbolzen 17.1 parallelen, waagerechten Achse Z–Z (Fig. 10) verschiebbar sind. Diese Riegelschieber 16.6 greifen in ihrer aus den Führungen vorgeschobenen Stellung wahlweise in jeweils zwei entsprechende, axial gegenüberliegende und symmetrisch gegenüber der vertikalen Längsmittelebene angeordnete Blindlöcher, z.B. 12.1 (Fig. 11) ein, die innenseitig in den Längswänden 12, 13 in einem Kreisbogen mit dem Mittelpunkt auf der erwähnten Achse Y–Y und mit einem Radius entsprechend dem Abstand zwischen dieser Achse Y–Y und der Achse Z–Z der Riegelschieber 16.6 angeordnet sind (in den Figuren ist nur die Anordnung der Blindlöcher 12.1 in der einen Längswand 12 sichtbar). Daher ist der Fensterrahmen 16.1 mit dem Glasfenster 16 in mehrere Stellungen mit unterschiedlichem Neigungswinkel verschwenkbar und mittels der Riegel 16.5 bzw. der Riegelschieber 16.6 unter deren Eingriff in die entsprechenden Blindlöcher z.B. 12.1 der Längswände der Kabine 10 in der gewählten Schwenkstellung feststellbar. In den Fig. 9 und 11 ist das Glasfenster 16 mit vollausgezogenen Linien in seiner minimal gegenüber der Vertikalen geneigten Schwenkstellung und mit gestrichelten Linien in seiner maximal geneigten Schwenkstellung dargestellt.

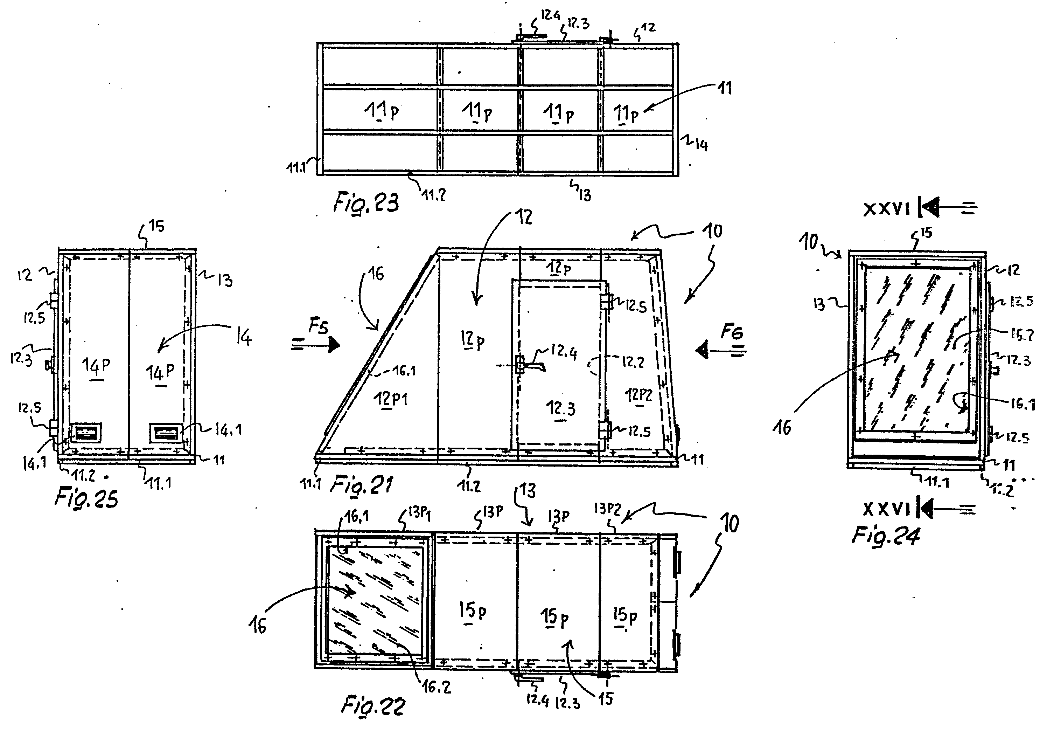

In der Längswand 12 der Kabine 10 ist eine rechteckige Durchgangsöffnung 12.2 (Fig. 1, 9, 10) vorgesehen, die mittels eines entsprechend bemessenen Außentürblattes 12.3 mit Riegelgriff 12.4 verschließbar ist. Dieses Türblatt ist mittels Scharniere 12.5 mit senkrecht gerichteten Scharnierbolzen, deren Scharnierblätter außen an der Längswand 12 und am Türblatt 12.3 befestigt sind, in Offen- bzw. Schließstellung schwenkbar (Fig. 1, 17, 18). Um bei geschlossener Tür eine gegen Eindringen von Luft, Staub, Wasser und andere Witterungseinflüsse sichere Abdichtung zu erzielen, sind zwischen dem Türblatt 12.3 und der Wandöffnung 12.2 dem Umfang entlang verlaufende Abdichtungsmittel 12.6 mit rohrförmigem – bei offener Tür 12.3 etwas vorstehendem – Profil 12.7 aus elastisch nachgiebigem Material an der Außenseite der Öffnung 12.2 angebracht (Fig. 17). Das rohrförmige Profil 12.7 dieser Abdichtungsmittel 12.6 wird beim Schließen des Türblattes 12.3 elastisch verformt und bewirkt unter dichter Anlage gegen die Innenseite des Türblattumfangs die gewünschte Abdichtung (Fig. 18). Bei dem dargestellten Ausführungsbeispiel bestehen die rohrförmigen Abdichtungsmittel 12.6 aus einem einfachen Streifen aus elastischem Material, z.B. einem Gummiband, das seiner Länge nach etwa nach Art eines Rohrschlauches umgebogen und mit seinen nach innen gerichteten Längsrändern mittels Schrauben am Umfang der Wandöffnung 12.2 der Kabine 10 befestigt wird.

Wie die Fig. 19 und 20 zeigen, sind Abdichtungsmittel 16.7 mit rohrförmigem Profil 16.8 in ähnlicher Ausführung wie die erwähnten Abdichtungsmittel 12.6 mittels durchgehender Schrauben mit Beilegscheibe innenseitig entlang der Seitenholme 16.9, 16.10 des schwenkbaren Fensterrahmens 16.1 unter dichter Anlage des rohrförmigen Profils 16.8 gegen die Innenseite der Längswände 12, 13 der Kabine 10 befestigt.

Wie außerdem aus den Fig. 9, 10 und insbesondere den Fig. 13, 14 hervorgeht, ist zwischen den Scharnierblättern 17 und der Innenseite der Bodenplatte 11 und der untersten Traverse 16.3 des Schwenkrahmens 16.1 ein Streifen 16.11 aus gummielastischem Material der gesamten Traverse 16.3 entlang nach Art eines langgestreckten flexiblen Scharniers zur Verbindung des Schwenkrahmens 16.1 mit der Bodenplatte 11 angebracht. Dieser elastische Streifen 16.11 ist außerdem z.B. mittels Schrauben gegenüber der Traverse 16.3 des Schwenkrahmens 16.1 und dem Bodenteil 11 in dem Zwischenraum zwischen den Scharnieren 17 (Fig. 10) befestigt, um eine satte Anlage an diese Teile seiner gesamten Länge entlang zu erzielen. Der elastische Streifen 16.11 bildet daher eine wirksame Andichtung zwischen den Anlenkungsteilen unter Mitwirkung mit den Scharnieren 17 während der Schwenkbewegung des Fensterrahmens 16.1. Wie besonders die Fig. 11 und 12 zeigen, sind außerdem zwischen der obersten Traverse 16.4 des Schwenkrahmens 16.1 und dem Flachdach 15 ebenfalls streifenförmige Abdichtungsmittel 16.12 aus flexiblem undurchläßigem Material eingesetzt. Dieser Abdichtungsstreifen 16.12 besitzt eine Breite, die etwa dem Abstand zwischen der freien Querseite des Daches 15 und der senkrechten Querebene, die im Inneren der Kabine 10 die um den größten Neigungswinkel eingeschwenkte Stellung des Fensterrahmens 16.1 begrenzt (mit gestrichelten Linien in Fig. 11 dargestellte Stellung) entspricht. Der Streifen 16.12 ist z.B. mittels Schrauben mit einem seiner Längsränder an die Unterseite des Daches 15 der gesamten Querbreite entlang befestigt. Dabei ist dieser Streifen gegenüber der freien Traverse des Daches 15 um einen im Vergleich zur Breite des Streifens 16.12 geringeren Abstand zurückgesetzt, während am anderen Längsrand der Streifen 16.12 z.B. mittels Schrauben an der Oberseite der obersten Traverse 16.4 des Schwenkrahmens 16.1 festgesetzt ist. Auf Grund dieser Anordnung begleitet der Abdichtungsstreifen 16.12 den Rahmen 16.1 in seiner Schwenkbewegung, ohne dabei diese Schwenkbewegung um die Achse Y–Y zu behindern, und bewirkt dadurch eine sichere Abdichtung gegen das Eindringen von Staub, Luft, Wasser u.a. Witterungseinflüssen zwischen der obersten Traverse 16.4 des Rahmens 16.1 und dem Dach 15. Anstatt eines derartigen gummielastischen Streifens kann selbstverständlich auch eine hinreichend starke, gegen Luft, Wasser und dergleichen undurchlässige Gewebedichtung eingesetzt werden.

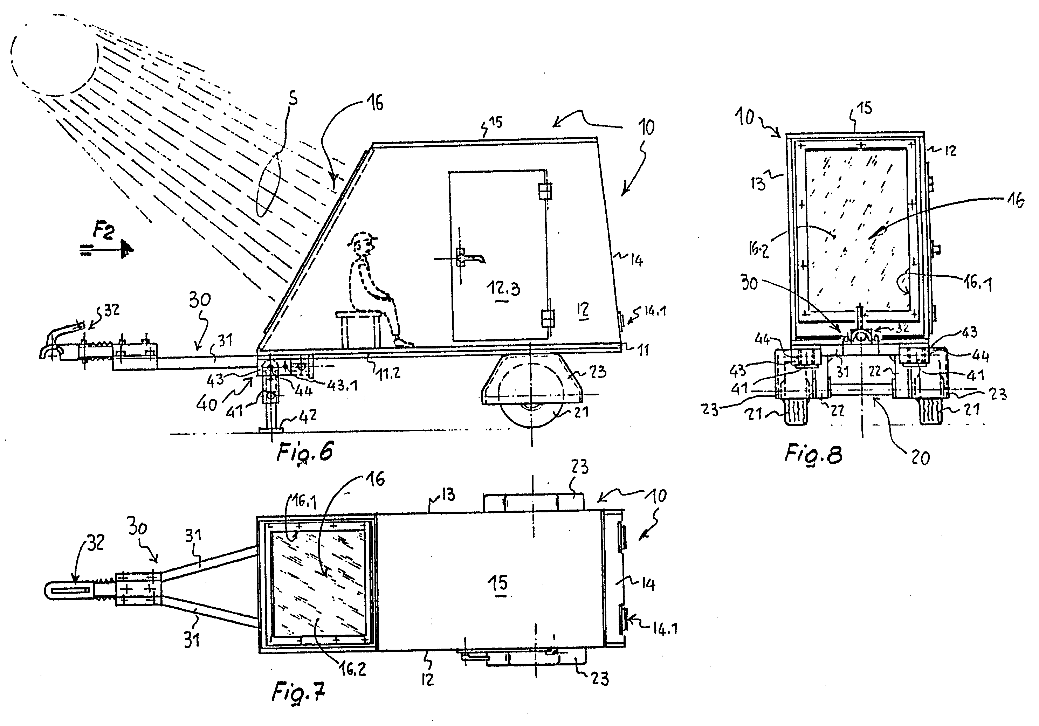

Zur steuerbaren Klimatisierung und Belüftung des Innenraumes der Kabine 10 sind in der Querwand 14 Belüftungshauben 14.1 (im dargestellten Beispiel der Fig. 5: zwei) vorgesehen. Diese Belüftungshauben 14.1 bestehen (Fig. 9, 15, 16) je aus einer schlitzartigen Öffnung 14.2, die durch ein entsprechendes Gitter 14.3 abgeschützt und in ihrer Öffnungsweite zur Regelung des Luftdurchflusses durch geeignete Mittel 14.4 von Hand einstellbar ist.

Diese Mittel 14.4 umfassen einen satt an der Querwand 14 in waagerechten Führungen 14.6 verschiebbaren Deckel 14.5 (Fig. 15, 16). In Fig. 16 sind diese Mittel 14.4 mit ausgezogenen Linien im geschlossenen Zustand dargestellt, in dem der Deckel 14.5 vollständig in seine Führungen 14.6 eingeschoben ist, so daß die Öffnung 14.2 vom Deckel verschlossen und jeder Luftdurchlaß unterbunden ist; mit gestrichelten Linien ist hingegen der Deckel 14.5 in seiner maximalen Auszugsstellung aus den Führungen 14.6 angedeutet, in der die Mittel 14.4 vollständig offen sind und der Luftdurchfluß durch die Öffnung 14.2 in vollem Umfang freigegeben ist.

Die Behandlungskabine 10 ist mit einer an sich bekannten, nicht dargestellten Innenbeleuchtung, die von einer Batterie mit elektrischem Strom versorgt wird, versehen. Außerdem kann die Kabine 10 mit in bekannter Weise angeordneten Trennwänden, die feststehend oder beweglich eingebaut sind, zum Abteilen der Kabine in mehrere Innenräume, z.B. auch zum Abtrennen eines Umkleideraumes, verwendet werden.

Die Fig. 6 bis 8 zeigen eine zweite Ausführungsform der erfindungsgemäßen thermisch-solaren Behandlungskabine, bei der zur leichteren Beförderung und Orientierung gegenüber den einfallenden Sonnenstrahlen S die Kabine 10 nach Art eines Anhängerfahrzeugs eine Fahrachse 20 mit bereiften Rädern 21 und ein vorderes, waagerecht ausgerichtetes steifes Gestell 30 aufweist. Dieses Gestell ist mit dem Bodenteil 11 fest verbunden und als Zuggabel zum Anschließen an ein (nicht dargestelltes) Zugfahrzeug ausgebildet. Außerdem besitzt diese Kabine 10 klappbare Stützböcke 40, die nach dem Aushängen der Kabine vom Zugfahrzeug die Kabine abstützen. Die Fahrachse 20 ist in bekannter Weise mit dem Bodenteil 11 in gegenüber dem Schwerpunkt der Last zurückversetzter Stellung unter Zwischenlage von gefederten Aufhängungen, z.B. Blattfedern 22, verbunden. Die bereiften Räder 21 sind zum Teil durch Kotflügel 23, die am Bodenteil 11 befestigt sind, abgeschützt. Das vordere steife Gestell 30 besitzt nach Art üblicher Zuggabeln für Anhänger wenigstens eine (nicht dargestellte) Traverse, die an der Unterseite der Bodenplatte 11 befestigt ist, und zwei Holme 31, die mit dieser Traverse fest verbunden sind und gabelförmig auseinanderlaufend die Zuggabel mit der Kabine 10 verbinden. Dabei sind diese Traverse und die Holme 31 des Gestells 30 durch feste Metallprofile, z.B. Vierkantprofile, gebildet. Die Holme 31 des Gestells 30 sind am freien Ende mit einem Zughaken 32 zum Anschließen an das Zugfahrzeug versehen. In einer abgewandelten Ausführung kann das vordere Gestell

30 am Bodenteil 11 angelenkt und in einer senkrechten Längsebene der Kabine 10 verschwenkbar sein.

Die klappbaren Abstützböcke 40 sind durch zwei Schwenkarme 41 mit Standfüßen 42 gebildet und mit ihrem oberen Ende an symmetrisch von der Mittellinie der Kabine beabstandeten, nahe dem vorderen Ende des Bodenteils 11 unter demselben angeordneten Stützgabeln 43 angelenkt. Die Anlenkung der Schwenkarme 41 in den Stützgabeln 43 erfolgt mittels waagerecht und quer zur Kabine 10 derart angeordneten Bolzen 44, daß die Arme 41 in vertikalen Längsebenen der Kabine 10 schwenkbar sind. Jeder Schwenkarm besteht aus zwei teleskopartig ineinander verschiebbaren Rohrstücken mit jeweils mehreren radial und koaxial angeordneten Löchern zum Durchstecken eines Feststellstiftes, um eine mehr oder weniger große Ein- bzw. Auszugslänge der teleskopartig ineinander verschiebbaren Rohrstücke eines jeden Schwenkarmes 41 einzustellen. Dadurch können die in Gebrauchsstellung geklappten Schwenkarme 41 abhängig von der Beschaffenheit des Standbodens in ihrer Länge eingestellt werden. Außer Gebrauch sind die Schwenkarme 41 in gegen die Unterseite der Bodenplatte 11 gekippter Stellung mittels ihrer erwähnten Feststellstifte, die dabei auch in entsprechend in den Stützgabeln 43 vorgesehenen Löchern 43.1 (Fig. 6) mit zu den Anlenkungsbolzen 44 paralleler Achse eingreifen, festsetzbar. Im übrigen ist der Aufbau der Kabine 10 gemäß dieser Ausführungsform durchaus gleichartig, wie mit Bezug auf die erste Ausführungsform geschildert und dargestellt, ausgebildet.

Die Fig. 21 bis 37 zeigen eine dritte Ausführungsform der thermisch-solaren Behandlungskabine gemäß der Erfindung, worin die Kabine 10 aus in einfacher Weise zerlegbaren und wieder zusammensetzbaren Teilen besteht.

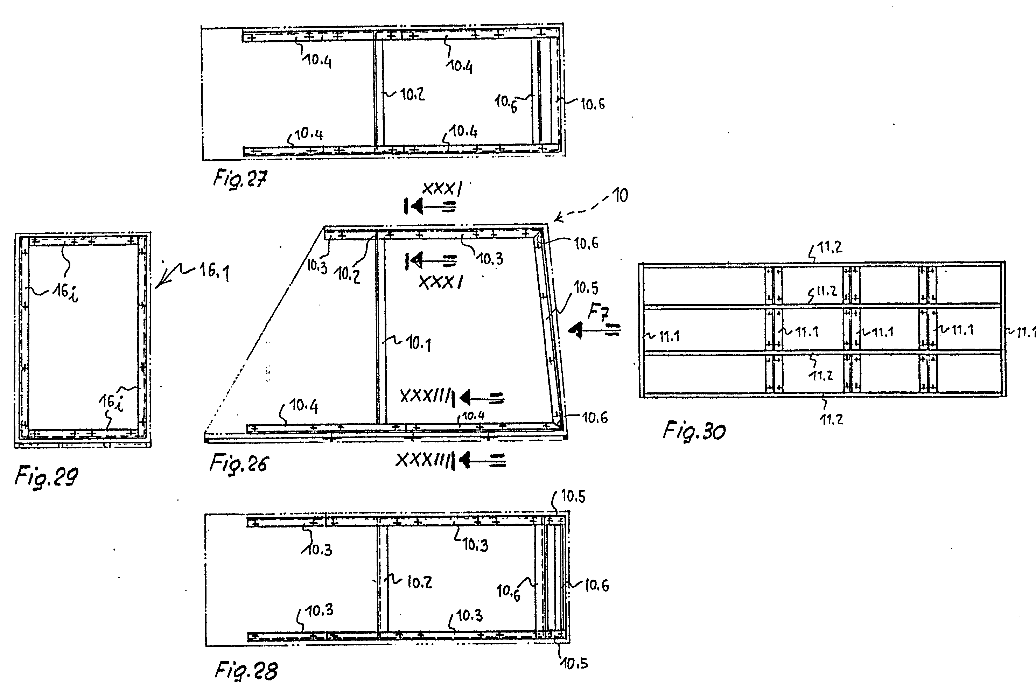

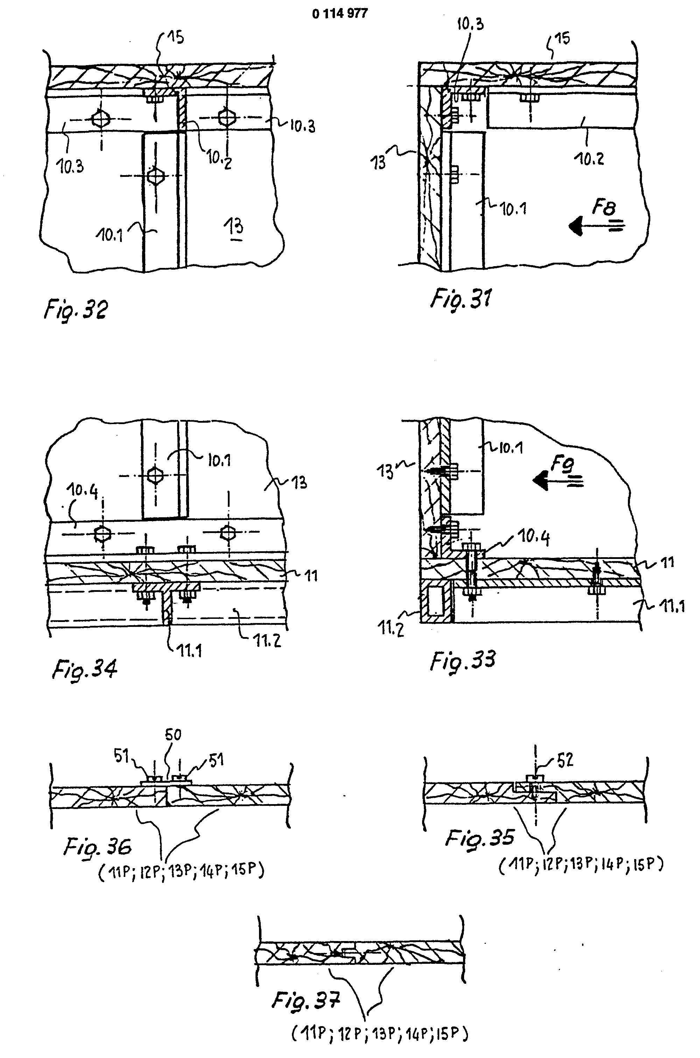

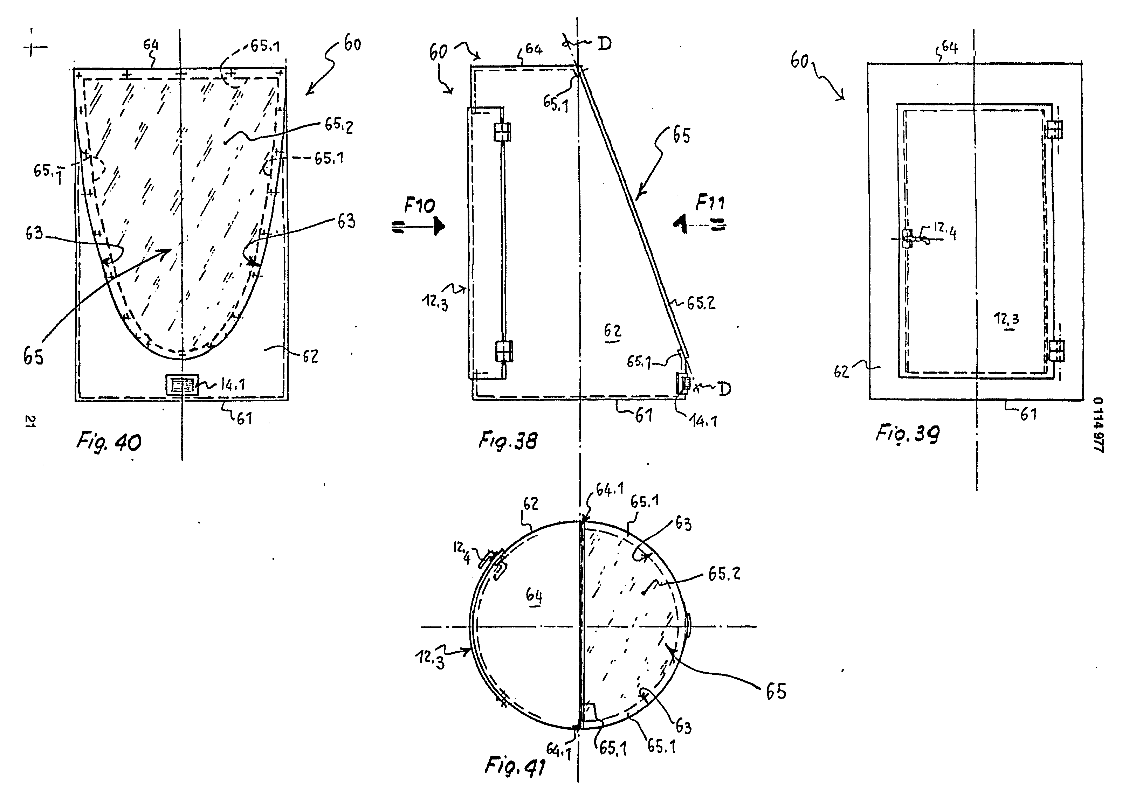

Mit Bezugnahme auf die Fig. 21 bis 25 sind die Bodenplatte 11, die beiden Längswände 12, 13, die Querwand 14 und das Dach 15 durch aneinander ansetzbare rechteckige Plattenteile 11P, 12P, 13P, 14P und 15P gebildet; für die Längswände 12, 13 sind außerdem je ein dreieckiger Plattenteil 12P1, 13P1 an einem Ende und je ein trapezförmiger Teil 12P2, 13P2 am anderen Ende vorgesehen. Diese Plattenteile werden entweder durch Stoßverbindung mittels Laschen 50 und Schrauben 51 (Fig. 36), oder durch Formschluß mittels Überblattung und Schrauben 52 (Fig. 35), oder durch Formschluß mittels Verzapfung bzw. Spundung (Fig. 37) zusammengebaut, wobei eine Verbindung durch Formschluß den Vorteil einer besseren Abdichtung mit sich bringt. Dementsprechend sind gemäß Fig. 26 bis 28 und 30 das aus Stützen 10.1, Traversen 10.2 und Holmen 10.3 bestehende Traggerippe für die Kabine 10 sowie der aus Traversen 11.1 und Holmen 11.2 bestehende Versteifungsrahmen für die Bodenplatte 11 durch lösbare Verbindung von Metallprofilen untereinander und mit den einzelnen Plattenteilen unter Verwendung von Schrauben mit oder ohne Muttern zusammengebaut (Fig. 31 bis 34). Bei dem vorliegenden Beispiel ist zur stärkeren Versteifung des Traggerippes ein zusätzlicher, aus Stützen 10.5 und Traversen 10.6 bestehender Rahmen für die Querwand 14 vorgesehen. Dieser Zusatzrahmen ist mit den Holmen 10.3 sowie den Holmen 10.4, die zur weiteren Verbindung der Längswände 12, 13 mit der Bodenplatte 11 dienen, lösbar verbunden und besteht ebenfalls aus Metallprofilen, die untereinander und mit den erwähnten Aufbauteilen lösbar zusammengefügt sind. Ein Rahmen 16i aus Metallprofilen verstärkt auch den Schwenkrahmen 16.1 des Glasfensters 16 (Fig. 29). Im übrigen sind die einzelnen Bauteile der Kabine 10 gemäß dieser Ausführungsform hinreichend klar aus den Figuren ersichtlich, so daß sich eine weitere Beschreibung derselben erübrigt.

Eine vierte Ausführungsform der erfindungsgemäßen Behandlungskabine ist in den Fig. 38 bis 41 gezeigt und in ihrem Ganzen mit 60 bezeichnet. Die Kabine 60 ist nach Art eines geraden Kreiszylinders, der durch eine schräge Ebene geschnitten ist (Schnittfläche D–D in Fig. 38), ausgebildet. Diese Schnittfläche geht von einem Durchmesser der oberen Grundfläche aus und erstreckt sich bis kurz oberhalb der unteren Grundfläche des Zylinders (Fig. 38 und 41). Die Überschneidung dieser Schnittebene mit der Mantelfläche des Zylinders ergibt ein etwa halbelliptisches Profil (Fig. 40). Im einzelnen besitzt die Kabine 60 eine kreisrunde Bodenplatte 61 und eine senkrechte Mantelwand 62 mit einer Fensteröffnung 63, die sich im wesentlichen über die ganze Höhe der Kabine erstreckt und, wie erwähnt, eine etwa halbelliptische Form aufweist. Ein steifes, halbkreisförmiges Flachdach 64 ist mit der Mantelwand und mit der die erwähnte Fensteröffnung 63 in der Mantelwand 62 oben begrenzenden Durchmesserseite 64.1 fest verbunden (Fig. 41). Die zwischen der Mantelwand 62 und dem Dach 64 gebildete Fensteröffnung ist ihrem Umfang entlang von einem durchgehenden Rahmen 65.1 umfaßt, der flanschartig in die Öffnung vorsteht und an dem eine entsprechend geformte Fensterverglasung 65.2 befestigt ist. Somit weist auch diese Ausführungsform ein entsprechend groß bemessenes, oben nach innen geneigtes Fenster 65 auf. Dieses Fenster 65 kann auch gemäß einer Abwandlung einen Schwenkrahmen aufweisen, der an der Durchmesserseite 64.1 des Daches 64 angelenkt und seinem Umfang entlang mit geeigneten Abdichtungsmitteln versehen ist.

Es ist von Vorteil, wenn erfindungsgemäß die Kabine ein mit einer Linsenverglasung bestücktes Schwenkfenster aufweist, damit die einfallenden Sonnenstrahlen durch Veränderung des Neigungswinkels des Fensters zweckmäßig fokussiert und dadurch die Wirkungen der Sonnenbestrahlung behandlungsgerecht gesteuert werden können.

Die Abdichtungsmittel 16.7 (s. S. 9) bewirken einen dichten Abschluß gegen Luft, Staub, Wasser und dergleichen zwischen den Seitenholmen 16.9, 16.10 des Schwenkrahmens 16.1 für das Glasfenster 16 und den Seitenwänden 12, 13 der Kabine 10 und dies auch bei der Schwenkbewegung des Rahmens 16.1 um die Achse Y–Y durch die gleitende Berührung ihres schlauchförmigen Dichtkörpers 16.8 gegen die Innenfläche der anliegenden Seitenwand trotz der hohen elastischen Verformbarkeit dieses schlauchförmigen Dichtkörpers.

### Patentansprüche

1. Thermisch-solare Behandlungskabine (10) zur Behandlung von Personen und/oder Tieren, die sich im Innenraum der Behandlungskabine befinden, durch Einwirkung der Sonnenstrahlen, insbesondere für solare Sauna-Bäder, mit Belüftungsöffnungen (14.2) und einem von unten nach oben gegen das Innere der Kabine (10) geneigten Glasfenster (16), dadurch gekennzeichnet, daß zur rationellen Ausnützung der Wirkung der Lichtstrahlen, insbesondere der ultravioletten und infraroten Sonnenstrahlen, unter steuerbaren Bedingungen und mit hohem Wirkungsgrad:

a) das Glasfenster (16) sich auf einer Kabinenwand im wesentlichen über die ganze Höhe der Kabine erstreckt,

b) zwischen dem Rahmen des Glasfensters (16) und dem Kabinenkörper ein allseitig dichter Verschluß vorgesehen ist,

c) die Öffnungsweite der Belüftungsöffnungen (14.2) mit Hilfe von verstellbaren Abschlußmitteln (14.4) zur Regelung des Luftdurchflusses einstellbar ist.

2. Thermisch-solare Behandlungskabine nach Anspruch 1, dadurch gekennzeichnet, daß die Abschlußmittel der Belüftungsöffnungen (14.2) aus je einem in Führungen (14.6) verschiebbaren Deckel (14.5) bestehen.

3. Thermisch-solare Behandlungskabine nach Anspruch 1 oder 2, dadurch gekennzeichnet, daß zwischen dem um eine waagerechte Achse (Y–Y) schwenkbar am Kabinenkörper gelagerten Rahmen (16.1) des Glasfensters (16) und dem Kabinenkörper allseitig Abdichtungsmittel (16.11, 16.12, 16.8) vorgesehen sind, die die Schwenkbewegung des Fensterrahmens (16.1) zulassen und eine sichere Abdichtung bei dieser Schwenkbewegung bewirken.

4. Thermisch-solare Behandlungskabine nach Anspruch 3, dadurch gekennzeichnet, daß zwischen der unteren und oberen Traverse (16.3, 16.4) des Fensterrahmens (16.1) und dem Kabinenboden (11) bzw. dem Kabinendach (15) je ein Abdichtungsstreifen (16.11, 16.12) aus flexiblem, undurchlässigem Material vorgesehen ist, der sich über die ganze Fensterbreite erstreckt und einerseits am Fensterrahmen (16.1) und andererseits am Kabinenboden (11) bzw. am Kabinendach (15) befestigt ist.

5. Thermisch-solare Behandlungskabine nach Anspruch 3, dadurch gekennzeichnet, daß an den Seitenholmen (16.9, 16.10) des Fensterrahmens (16.1) Abdichtungsmittel (16.8) mit rohrförmigem Profil befestigt sind, die gleitend gegen die Innenseite der benachbarten Seitenwände (12, 13) der Kabine (10) anliegen.

6. Thermisch-solare Behandlungskabine nach einem der Ansprüche 1 bis 5, dadurch gekennzeichnet, daß die Behandlungskabine (60) als gerader Hohlzylinder ausgebildet ist, dessen Mantelwand (62) durch eine schräge, von einem Durchmesser der oberen Grundfläche bzw. Dach (64) ausgehende und sich bis kurz vor der unteren Grundfläche bzw. Bodenplatte (61) des Zylinders erstreckende Schnittebene geschnitten ist und der ein entsprechend dieser Schnittebene geneigtes Glasfenster (65), das die durch die erwähnte Schnittebene gebildete Öffnung abschließt, aufweist.

### Claims

1. Thermal solar treatment cabin (10) for treatment of persons and/or animals, that are in the inner space of the treatment cabin, through the action of solar radiation, especially for solar sauna baths, with ventilation openings (14.2) and a glass window (16) sloping from below to above towards the inside of the cabin (10), characterized in that for rational utilization of the action of the light radiation, especially the ultraviolet and infrared solar radiation, under controllable conditions and with high efficiency:

a) the glass window (16) extends in a cabin wall essentially over the total height of the cabin,

b) between the frame of the glass window (16) and the cabin body a tight seal is provided on all sides,

c) the width of opening of the ventilation openings (14.2) can be set with the help of adjustable closing devices (14.4) for regulating the air flowthrough.

2. Thermal solar treatment cabin according to Claim 1, characterized in that the closing devices for the ventilation openings (14.2) each consists of a cover (14.5) slideable in guides (14.6).

3. Thermal solar treatment cabin according to Claim 1 or Claim 2, characterized in that between frame (16.1) of the glass window (16), the frame (16.1) being pivotably fastened to the cabin body about a horizontal axis (Y–Y), and the cabin body, sealing devices (16.11, 16.12, 16.8) are provided on all sides, which allow a pivoting movement of the window frame (16.1) and effect a secure sealing with this pivoting movement.

4. Thermal solar treatment cabin according to Claim 3, characterized in that between the lower and upper cross pieces (16.3, 16.4) of the window frame (16.1) and the cabin base (11) or the cabin roof (15) respectively in each case a sealing band (16.11, 16.12) of flexible, impermeable material is provided, which extends over the whole window width and is fastened on one side to the window frame (16.1) and on the other side to the cabin base (11) or the cabin roof (15) respectively.

5. Thermal solar treatment cabin according to Claim 3, characterized in that sealing devices (16.8) with a tubular profile are fastened on the side beams (16.9, 16.10) of the window frame (16.1), which slideably lie against the inner sides of the neighbouring side walls (12, 13) of the cabin (10).

6. Thermal solar treatment cabin according to one of Claims 1 to 5, characterized in that the treatment cabin (60) is built as an upright hollow cylinder, the covering wall (62) of which is cut by a sloping cutting plane starting from one diameter of the upper basic surface or roof (64) and extending to just before the lower basic surface or base plate (61) respectively of the cylinder, and which has a glass window (65) with a slope corresponding to this cutting plane which closes the opening formed by the said cutting plane.

## Revendications

1. Cabine thermique solaire (10) pour le traitement, sous l'action des rayons solaires, de personnes et/ou d'animaux situés dans l'espace interne de la cabine de traitement, en particulier pour des bains solaires de sauna, présentant des orifices de ventilation (14.2) et une baie vitrée (16) inclinée, de bas en haut, vers l'intérieur de la cabine, caractérisée par le fait que, pour l'exploitation rationnelle de l'action des rayons lumineux, en particulier des rayons solaires ultraviolets et infrarouges, dans des conditions commandables et avec un fort degré d'efficacité:

a) la baie vitrée (16) s'étend sur une paroi de la cabine, pour l'essentiel sur toute la hauteur de cette cabine,

b) une obturation étanche de toutes parts est prévue entre le châssis de la baie vitrée (16) et le corps de la cabine,

c) la largeur d'ouverture des orifices de ventilation (14.2) peut être ajustée, à l'aide de moyens obturateurs réglables (14.4), en vue du réglage du débit d'air.

2. Cabine thermique solaire de traitement selon la revendication 1, caractérisée par le fait que les moyens obturateurs des orifices de ventilation (14.2) consistent en un couvercle respectif (14.5), pouvant coulisser dans des guides (14.6).

3. Cabine thermique solaire de traitement selon la revendication 1 ou 2, caractérisée par le fait qu'il est prévu de toutes parts, entre le corps de la cabine et le châssis (16.1) de la baie vitrée (16) monté pivotant sur le corps de la cabine autour d'un axe horizontal (Y–Y), des moyens d'étanchement (16.11, 16.12, 16.8) qui autorisent le mouvement pivotant du châssis (16.1) et confèrent une étanchéité sûre lors de ce mouvement pivotant.

4. Cabine thermique solaire de traitement selon la revendication 3, caractérisée par le fait qu'il est prévu, entre les traverses inférieure et supérieure (16.3, 16.4) du châssis (16.1) de la fenêtre et le fond (11) de la cabine ou, respectivement, le toit (15) de cette cabine, une bande respective d'étanchement (16.11, 16.12) en un matériau flexible imperméable, qui s'étend sur toute la largeur de la baie et est fixée, d'une part, audit châssis (16.1) et, d'autre part, respectivement au fond (11) de la cabine ou au toit (15) de cette cabine.

5. Cabine thermique solaire de traitement selon la revendication 3, caractérisée par le fait que des moyens d'étanchement (16.8) à profil tubulaire, fixés aux longerons latéraux (16.9, 16.10) du châssis (16.1) de la fenêtre, sont appliqués de manière coulissante contre la face interne des parois latérales voisines (12, 13) de la cabine (10).

6. Cabine thermique solaire de traitement selon l'une des revendications 1 à 5, caractérisée par le fait que cette cabine de traitement (60) est réalisée sous la forme d'un cylindre droit creux, dont la paroi d'enveloppement (62) est coupée par un plan de coupe incliné, qui part d'un diamètre de la surface de base supérieure ou respectivement du toit (64), et s'étend jusqu'à une courte distance avant la surface de base inférieure ou, respectivement, le panneau de fond (61) du cylindre, ce cylindre présentant une fenêtre vitrée (65) qui est inclinée d'une manière correspondant à ce plan de coupe, et qui ferme la baie formée par le plan de coupe précité.

0 114 977

Fig. 6

Fig. 7

Fig. 8

Fig. 9

Fig. 10

Fig. 11

Fig. 12

Fig. 18

Fig. 20

Fig. 19

Fig. 16

Fig. 15

Fig. 17

Fig. 13

Fig. 14

0 114 977

Fig. 23

Fig. 25

Fig. 21

Fig. 22

Fig. 24

0 114 977

Fig.27

Fig.29

Fig.26

Fig.30

Fig.28

10.4   10.4   10.2   10.6   10.6

16.1   16i   16i   17

XXXI   10   10.6   10.3   10.2   10.3   10.5   F7   10.1   XXXI   XXXIII   10.4   10.4   10.6   XXXIII

11.2   11.2   11.1   11.1   11.1   11.1   11.1   11.2   11.2

10.5   10.3   10.3   10.2   10.6   10.6   10.3   10.3   10.5

0 114 977

Fig. 32

Fig. 31

Fig. 34

Fig. 33

Fig. 36
( 11P ; 12P ; 13P ; 14P ; 15P )

Fig. 35
( 11P ; 12P ; 13P ; 14P ; 15P )

Fig. 37
( 11P ; 12P ; 13P ; 14P ; 15P )

Fig. 40

Fig. 38

Fig. 39

Fig. 41

0 114 977